# EUROPEAN PATENT SPECIFICATION

(11) **EP 4 340 766 B1**
(45) Date of publication and mention of the grant of the patent: **25.03.2026**
(21) Application number: 22727941.1
(22) Date of filing: 16.05.2022
(51) Int. Cl.: A61B 18/24

(54) **DEVICES FOR ENABLING ACTIVE MONITORING AND COMMUNICATIONS BETWEEN MEDICAL FIBER OPTIC CATHETERS AND MEDICAL LASER LIGHT SYSTEMS**
VORRICHTUNGEN ZUR AKTIVEN ÜBERWACHUNG UND KOMMUNIKATION ZWISCHEN MEDIZINISCHEN GLASFASERKATHETERN UND MEDIZINISCHEN LASERLICHTSYSTEMEN
DISPOSITIFS POUR PERMETTRE UNE SURVEILLANCE ACTIVE ET DES COMMUNICATIONS ENTRE DES CATHÉTERS MÉDICAUX À FIBRE OPTIQUE ET DES SYSTÈMES MÉDICAUX DE LUMIÈRE LASER

(30) Priority: 18.05.2021 US 202163189746 P
(43) Date of publication of application: 27.03.2024
(73) Proprietor: Koninklijke Philips N.V., 5656 AG Eindhoven (NL)
(72) Inventor: SCHINKEL, Jeffrey, 5656 AG Eindhoven (NL); GRACE, Kenneth Peter, 5656 AG Eindhoven (NL); ANDERSON, Michael, 5656 AG Eindhoven (NL); CEZO, James David, 5656 AG Eindhoven (NL)
(74) Representative: Philips Intellectual Property & Standards
(86) International application number: PCT/EP2022/063142
(87) International publication number: WO 2022/243224

(56) References cited:
- WO-A1-2014/158688
- US-A- 4 919 508
- US-A- 5 400 428

## Description

### FIELD

The following relates generally to the catheter arts, catheter coupling arts, catheter detection arts, and related arts.

### BACKGROUND

Fiber optic catheters are used in a range of therapies, such as for performing atherectomies and other types of vascular procedures. In an atherectomy performed using a fiber optic catheter, the catheter is inserted into a large blood vessel and the tip is moved to a target location, and then an optical fiber or fiber bundle of the catheter carries high intensity light from a light source such as an excimer light source to the target location to ablate or otherwise remove atherosclerosis from the blood vessel at the target location. Similar therapies are sometimes also performed in veins. The catheter is usually a single-use medical accessory that attaches to a light source providing the laser light via a detachable coupling. The parameters of the laser light provided by the light source should be matched to optical transmission characteristics of the attached fiber optic catheter. To automate the matching process, it is advantageous for the light source to automatically recognize the catheter type of the fiber optic catheter when it is attached to the light source. In this context, the technical teaching provided by WO2014/158688 Al is acknowledged.

To this end, in some existing fiber optic catheter systems, a source (e.g., an excimer) laser-side coupler includes a bank of N mechanical switches. A mating catheter-side coupler includes molded pins that depress zero, all, or some subset of the N mechanical switches when the two couplers are mated together. Different types of catheters will have different subsets of molded pins. This approach conveys an N bit value to the laser, so that 2^{N} different types of catheters can be distinguished. Based on the type of catheter thusly identified, the laser can automatically configure the fluence rate, repetition rate, cycle time, and/or other operating parameters appropriate for that type of catheter using a look-up table that stores the operating parameters for each catheter type.

The following discloses certain improvements to overcome these problems and others.

### SUMMARY

The invention is defined in the appended set of claims. In some embodiments disclosed herein, a source-side coupler is provided for injecting light from an associated light source into an associated fiber optic catheter via a connection of the source-side coupler with a catheter-side coupler of the associated fiber optic catheter. The source-side coupler includes an optical output aperture configured to inject light from the associated light source into the associated fiber optic catheter via the connection. A plurality of mechanical switches is configured to be engaged by pins of the catheter-side coupler during the connection, and at least two electrical lines. An electronic processor is programmed to receive information from the associated fiber optic catheter via the connection by: electrically receiving the information if the connection establishes an electrical connection between the electronic processor and the associated fiber optic catheter via the at least two electrical lines, or mechanically receiving the information based on which mechanical switches of the plurality of mechanical switches are engaged by pins of the catheter-side coupler if the connection does not establish an electrical connection between the electronic processor and the associated fiber optic catheter via the at least two electrical lines.

In some embodiments disclosed herein, a fiber optic catheter apparatus includes a catheter including an optical fiber or optical fiber bundle terminating at an optical output aperture. A catheter-side coupler includes a plurality of electrically conductive pins. An electronic processor is programmed transmit information about the fiber optic catheter apparatus to an associated light source in response to an electrical connection of the catheter-side coupler with a source-side coupler of the associated light source via the electrically conductive pins. A physical configuration of the plurality of electrically conductive pins encodes at least a portion of the information about the fiber optic catheter apparatus.

In some embodiments disclosed herein, a light system for injecting light into a fiber optic catheter includes a light source. A fiber optic catheter apparatus includes a catheter including an optical fiber or optical fiber bundle terminating at an optical output aperture. A catheter-side coupler includes a plurality of electrically conductive pins. A fiber optic catheter electronic processor is disposed in the catheter-side coupler and programmed transmit information about the fiber optic catheter apparatus to the light source. A physical configuration of the plurality of electrically conductive pins encodes at least a portion of the information about the fiber optic catheter apparatus. A source-side coupler is configured to inject light from the light source into the fiber optic catheter apparatus via a connection of the source-side coupler with the catheter-side coupler. The source-side coupler includes an optical coupler configured to inject light from the light source into the fiber optic catheter via the connection. A plurality of mechanical switches is configured to be engaged by the electrically-conductive pins of the catheter-side coupler during the connection, and at least two electrical lines. A source-side electronic processor is programmed to receive information from the fiber optic catheter via the connection by electrically receiving the information if the connection establishes an electrical connection between the source-side electronic processor and the fiber optic catheter via the at least two electrical lines, or mechanically receiving the information based on which mechanical switches of the plurality of mechanical switches are engaged by pins of the catheter-side coupler if the connection does not establish an electrical connection between the electronic processor and the fiber optic catheter via the at least two electrical lines.

One advantage resides in determining a type of catheter based on a configuration of an engagement between a source side coupler and a catheter coupler.

Another advantage resides in determining a type of catheter based on a configuration of an engagement between a source side coupler and a catheter coupler based on an electrical connection between the source side coupler and the catheter coupler.

Another advantage resides in determining a type of catheter based on an electrical connection as noted above, but alternatively based on a mechanical connection between the source side coupler and the catheter coupler if the catheter or light source are not designed to employ the electrical connection.

Another advantage resides in determining operating parameters of catheter based on a configuration of an engagement between a source side coupler and a catheter coupler.

Another advantage resides in determining when a catheter has been used and issuing a notification regarding a used catheter.

Another advantage resides in measuring an output energy of a laser from a light source through a catheter.

Another advantage resides in digitizing analog catheter measurements to reduce noise in the measurements.

Another advantage resides in measuring a temperature measurement of an ambient environment and compensating for the temperature during an intravascular procedure.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically illustrates a catheter light system in accordance with the present disclosure.
FIGURE 2 diagrammatically illustrates a coupler of the system of the system of FIGURE 1.
FIGURE 3 diagrammatically illustrates another coupler of the system of the system of FIGURE 1.
FIGURE 4 diagrammatically illustrates the coupler of FIGURE 2 and the coupler of FIGURE 3 engaged with each other.
FIGURE 5 diagrammatically illustrates a catheter method using the system of FIGURE 1-4.

### DETAILED DESCRIPTION

It is recognized herein that there can be some deficiencies in the approach of employing mechanical pins and mating switches to identify the catheter type. First, the number of different types of lasers that can be distinguished is 2^{N}, and for a reasonable number of mechanical switches (e.g., N=6) this is a relatively small number (e.g., 2⁶=64 types). Second, updating the operating parameters for a particular type of catheter entails updating a lookup table storing the operating parameters for each catheter type at the laser. Also, if two catheters of the same type (as indicated by the molded pins) have different operating parameters, there is no way to accommodate this.

On the other hand, the deployed base of excimer lasers that use the existing mechanical switches/molded pins approach is large. While a fiber optic catheter is typically a single-use device due to sanitation considerations, light sources of the excimer lasers can be used multiple times. Hence, it is recognized herein that it would be advantageous for any improved catheter to be backward compatible with existing (primarily excimer) light sources that are designed to identify catheter type based on mated molded pins.

The following discloses an approach for providing a "smart" catheter which is also backward compatible with the existing mechanical switches-based laser-side coupler. The disclosed approach adds electronics to the catheter-side coupler and replaces the molded plastic pins with electrically conductive metal pins (either by wholesale replacement or by providing molded plastic pins with electrically conductive coatings). The laser-side coupler still has the mechanical switches but also includes electrical wires attached to those switches. Hence, if a "dumb" catheter is plugged into a "smart" excimer laser (or vice versa) then the catheter-type identification works as usual, relying on the mechanical switches being depressed to convey information on the type of catheter being plugged into the laser.

However, if both the catheter and the laser are "smart", then the smart laser detects the presence of a smart catheter based on electrical signals conveyed by the catheter electronics via the metal pins. Once identified as a smart catheter, information stored in memory of the catheter electronics can be transmitted to the smart laser. This can be done in wired fashion using the metal pins, or can be done wirelessly, e.g., by reading an RFID tag of the smart catheter. Information can also optionally be conveyed from the laser to the catheter.

The following discloses numerous use cases for the smart catheter design. In one approach, the memory of the catheter electronics stores the operating parameter values for the catheter, and these are conveyed to the laser. This simplifies upgrading a particular catheter type, as it requires only storing the correct (updated) operating parameters in the memory of the catheter prior to shipment.

In some embodiments disclosed herein, a photodiode can be added to the catheter-side coupler that measures the laser power transmitted through the catheter-side coupler. This can be done by arranging the photodiode to measure light that is leaked from the side of the optical fiber and applying a suitable calibration to extract the actual laser power. Optionally, a temperature sensor can also be included in the catheter-side coupler to refine laser power measurement accuracy if the photodiode output is overly temperature-dependent. Furthermore, to reduce noise the catheter electronics can include an analog-to-digital (A/D) converter to digitize the power reading before sending it to the laser.

In some embodiments disclosed herein, since the catheter is a single-use disposable item, the catheter electronics can store in memory a "used" flag that is set once the catheter is used. This flag value is sent to the laser, and if the "used" flag is set then the laser will not operate. Similarly, if the laser detects a fault condition during a procedure, it can write diagnostic information to the memory of the catheter electronics, which can be used to diagnose the problem when the faulty catheter is sent back to the manufacturer for analysis.

In some embodiments disclosed herein, a validation code can be conveyed from the catheter electronics to the laser, that is used to validate the catheter for use with the laser.

As previously noted, there are relatively few pins on the catheter-side coupler (e.g., a maximum of N pins corresponding to the N mechanical switches of the laser-side coupler, where N=6 in some systems). This is somewhat limiting since two pins will be used to convey electrical power to the catheter electronics. Hence, the wired communication preferably uses a serial method such as Inter-Integrated Circuit (I2C) serial communication. Alternatively, as previously noted, wireless communication could be used.

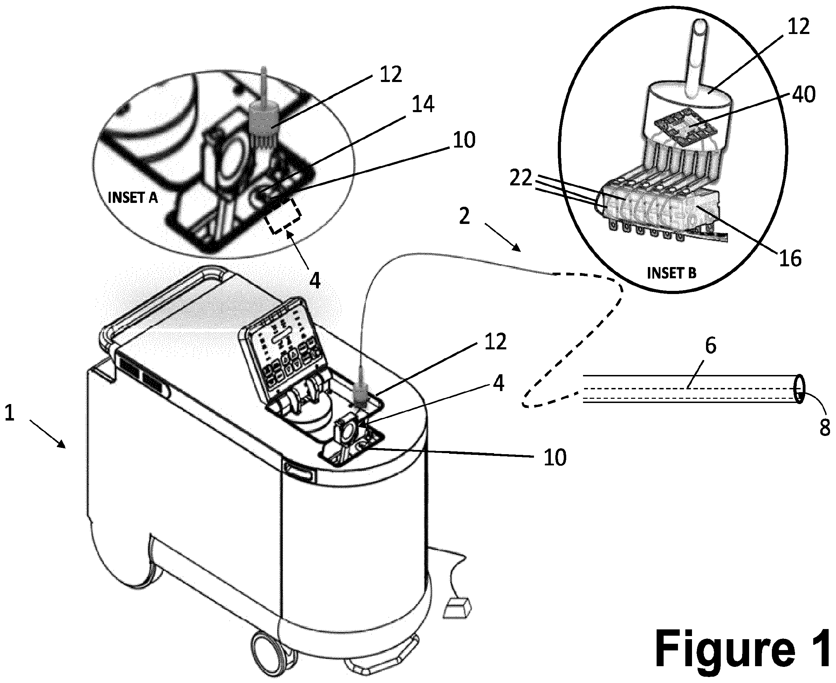

With reference to FIGURE 1, an illustrative light system **1** for use with a catheter **2** is shown. As shown in FIGURE 1, the light system includes one or more light sources **4** configured to supply or inject light into a fiber optic catheter **2.** The catheter **2** is diagrammatically shown with a dashed line indicating most of the length of the catheter, and the end of the catheter shown in enlarged diagrammatic fashion to illustrate that the catheter **2** includes an optical fiber **6** (which may be a fiber bundle) terminating at an optical output aperture **8.** The light system 1 also include a source-side (i.e., excimer) coupler **10** configured to engage or connect with a catheter-side coupler **12** of the catheter **2,** as best shown in INSET A of FIGURE 1. During an atherectomy or other vascular procedure, laser light from the light system 1 passes through the coupler **10** into a mating coupler **12** of the catheter **2** and is carried by the optical fiber or fiber bundle **6** and emitted at the optical output aperture **8** to ablate or otherwise remove atherosclerosis from a blood vessel at a target location.

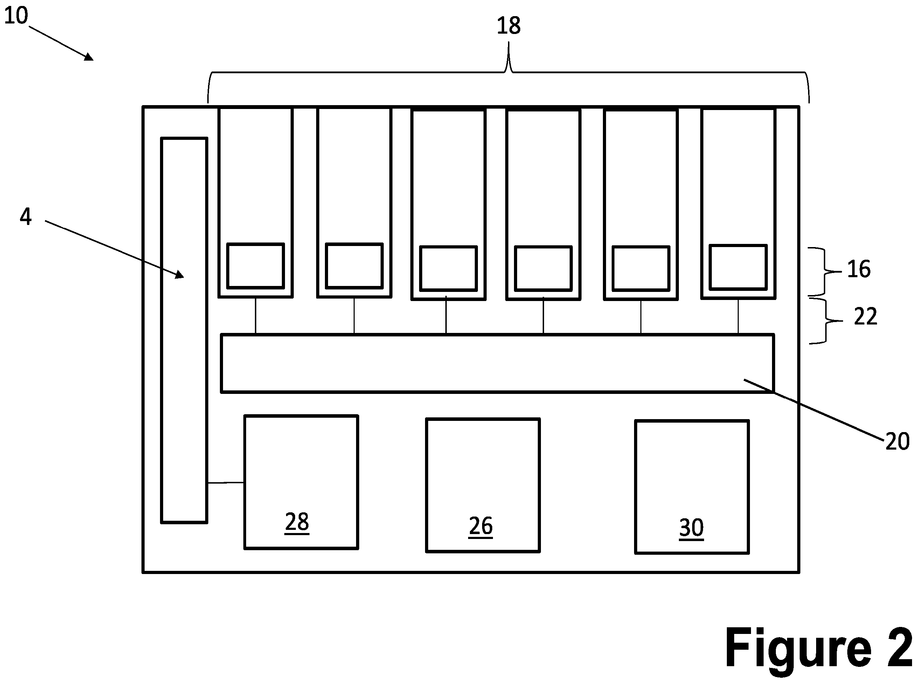

FIGURE 2 shows an example of the source-side coupler **10.** The source-side coupler **10** includes an optical output aperture **14** configured to inject light from the light source **4** into the fiber optic catheter **2** via the connection between the source-side coupler **10** and the catheter-side coupler **12.** The source-side coupler **10** also includes a plurality of mechanical switches **16,** in which each switch **16** is encased within a corresponding conductive socket **18** configured to be engaged by pins (not shown in FIGURE 2) of the catheter-side coupler **12** during the connection. Alternatively, as shown in Inset B, the switches **16** can be constructed as a bank of switches. A source-side electronic processor **20** is included in the source-side coupler **10** and is programmed to receive information from the fiber optic catheter **2** via the connection. To do so, in one example, two or more electrical lines **22** are included, and if the connection establishes an electrical connection between the source-side electronic processor **20** and the fiber optic catheter **2** via the at least two electrical lines **22.** Inset B of FIGURE 1 illustrates one non-limiting illustrative approach for providing the electrical lines **22.** Inset B also diagrammatically illustrates a catheter-side electronic processor **40** that suitably conveys the information to the source-side electronic processor **20.** However, this assumes that both the light source **1** and the catheter **2** are equipped (e.g., with the processors **20, 40** and associated electrical connectors) to transfer the information via the electrical connection. If, however, this is not the case, then the information is mechanically received by the source-side electronic processor **20** based on which mechanical switches of the plurality of mechanical switches **16** are engaged by pins of the catheter-side coupler **12** if the connection does not establish an electrical connection between the source-side electronic processor **20** and the fiber optic catheter **2** via the at least two electrical lines **22.** In a further example, the source-side coupler **10** includes a wireless radio receiver or transceiver **26,** and the source-side electronic processor **20** is programmed to electrically receive the information via the wireless radio receiver or transceiver **26.**

The source-side coupler **10** also includes a power supply **28** for power to be supplied thereto. In some examples, the power supply **28** can include a DC-to-DC converter which reduces the power supplied to the source-side coupler **10** from 12 Volts to 3.3 Volts. This is done in order to ensure that the source-side coupler **10** has plenty of power, even if the conductivity of the conductive sockets **18** degrades over time. In addition, the source-side coupler **10** also includes a serial communication circuit **30,** such as an I2C communication circuit, configured to communicate with a corresponding serial communication circuit (not shown in FIGURE 2) of the catheter-side coupler **12.** In some variant embodiments, the source-side coupler **10** can include printed traces (not shown) that do not connect with any pins of the catheter-side coupler **12,** but do make electrical contact with traces (not shown) elsewhere in the catheter-side coupler **12.** For example, electrical traces could be disposed on sidewalls of a socket and plug of a socket-and-plug connection arrangement. These electrical traces suitably replace the wires **22** shown in Inset B.

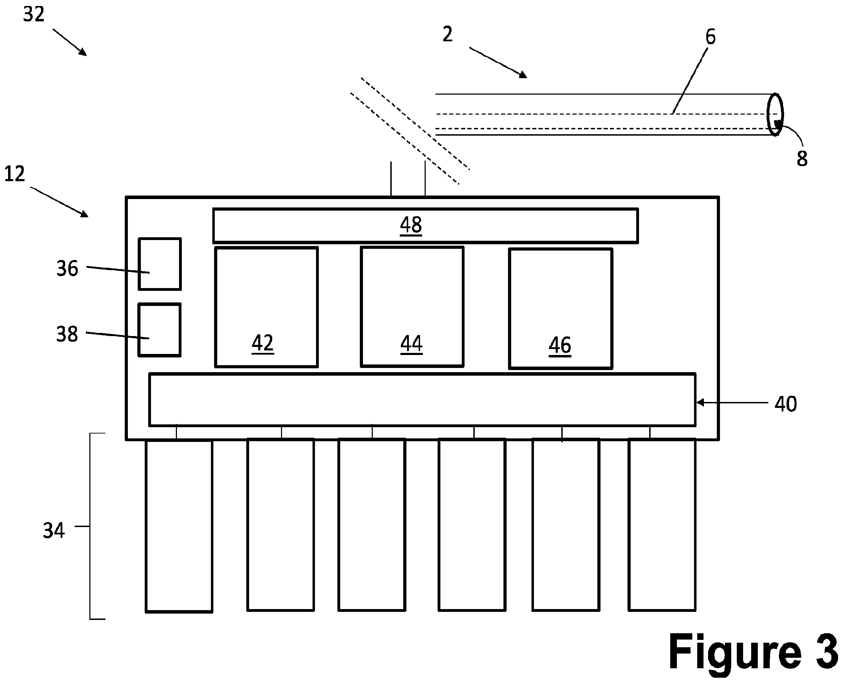

FIGURE 3 shows an example of a fiber optic catheter apparatus **32** that includes the catheter-side coupler **12** and the catheter **2.** As again shown in FIGURE 3, the catheter **2** includes the optical fiber or fiber bundle **6** terminating at the optical output aperture **8.** The catheter-side coupler **12** includes a plurality of electrically conductive pins **34** configured to engage corresponding mechanical switches **16** on the source-side coupler **10.** In some embodiments, a photodiode **36** is configured to measure a power of a laser beam from the light source **4** transmitted through the source-side catheter **10.** In some embodiments, a temperature sensor **38** is configured to measure an ambient temperature value of an ambient environment of the fiber optic catheter apparatus **32.** The photodiode **36** is configured to measure the power of the laser beam based on the measured ambient temperature value.

As shown in FIGURE 3, the catheter-side coupler **12** includes the catheter-side electronic processor **40** disposed therein and is programmed transmit information about the fiber optic catheter apparatus **32** to the light system **1** (e.g., the light source **4**) in response to an electrical connection of the catheter-side coupler **12** with the source-side coupler **10** via the electrically conductive pins **34.** At the same time, a physical configuration of the plurality of electrically conductive pins **34** encodes at least a portion of the information about the fiber optic catheter apparatus **32.** The portion of the information that can be encoded by the physical configuration of the pins **34** is typically a subset of the information that can be transmitted electrically, because the pins **34** can only encode an N-bit binary value where N is the number of pins **34.** As an example, for a six-pin connection, the 6-bit binary value can assume any one of 2^{N}=64 possible values. By contrast, the information that can be conveyed electrically is limited only by a data storage capacity of the catheter-side electronic processor **40** which can be on the order of kilobits, megabits, or more. Hence, the electrical transmission of the (complete) information is preferable over the mechanical transmission of (typically a subset of) the information; however, if the light source **4** to which the catheter **2** is connected is a model that is not designed to receive the information electrically then a sufficient subset of the information can be conveyed by the mechanical pin configuration (e.g. this can convey the catheter type, from which the light source **4** can extract operating parameters using a look-up table). For example, the catheter-side electronic processor **40** is configured to detect a configuration of the electrically conductive pins **34** that engage the mechanical switches **16** via signals generated by the electrical lines **22** of the engaged mechanical switches **16** to determine an identification of a catheter type of the catheter **2.** To do so, if the catheter-side coupler **12** includes, for example, six pins **34** (i.e., "pin 1" through "pin 6"), then a physical configuration of having pins 1, 2, and 5 with corresponding mechanical switches **16** can identify the catheter **2** as having a first type. Similarly, a physical configuration of having pins 1, 3, and 6 with corresponding mechanical switches **16** can identify the catheter **2** as having a second, different type. Moreover, the number of pins **34** engaged with the switches **16** (i.e., 2 engaged switches **16,** 4 engaged switches **4,** and so forth) can identify the type of the catheter **2.**

In some embodiments, the catheter-side electronic processor **40** is programmed to transmit the information about the fiber optic catheter apparatus **32** to the light system 1 via the electrically conductive pins **34.** To do so, a serial communication circuit **42,** such as an I2C communication circuit, is configured to communicate with the serial communication circuit **30** of the source-side coupler **10.** In a further example, the source-side coupler **10** includes a wireless radio receiver or transceiver **44,** and the catheter-side electronic processor **40** is programmed to transmit the information about the fiber optic catheter apparatus **32** to the light system **1** via the wireless radio receiver or transceiver **44.**

In some embodiments, the catheter-side coupler **12** includes a memory **46** that stores memory content, including at least a catheter type of the fiber optic catheter apparatus **32.** The transmitted information about the fiber optic catheter apparatus **32** includes the memory content. In some examples, the identification of the catheter type of the catheter **2** is transmitted to the memory **46** using a wired connection based on a number of mechanical switches **16** engaged by the electrically conducting pins **34.** In some examples, upon determination of the identification of the type of the catheter **2,** operating parameters of the catheter **2** are stored in the memory **46.** These operating parameters can include, for example, an identification of the catheter **2,** maximum fluence, minimum fluence, maximum rate, minimum rate, usage information, run time information and error information, among others. The memory **46** is illustrated as a separate component from the electronic processor **40;** however, the memory alternatively may be integrated with the electronic processor **40,** e.g. as a microprocessor with on-board memory.

As previously mentioned, the fiber optic catheter 2 is typically a single-use medical accessory due to sanitary considerations, e.g. the need to avoid cross-contamination between patients. In some embodiments, the catheter-side electronic processor **40** is programmed to set a flag indicating the catheter **2** has been used, which can be stored in the memory **46.** Advantageously, this can prevent a catheter **2** from being used multiple times. The transmitted information about the fiber optic catheter apparatus **32** includes the set flag. If the set flag is received by the source-side coupler **10,** then the source-side electronic processor **20** is programmed to prevent operation of the light source **4** from transmitting a laser beam. In another example, the catheter-side electronic processor **40** is configured to generate a fault condition flag indicative of when the catheter **2** has a fault condition, which can be stored in the memory **46.** The fault condition flag may be a multi-bit flag that encodes information about the fault condition. The faulty catheter **2** can then be shipped back to the manufacturer which can read out the content of the memory **46** including the fault condition flag for use in determining why the catheter **2** failed. In another example, the catheter-side electronic processor **40** is configured to generate a validation code indicative of whether the source-side catheter **10** has been validated for use, which can be stored in the memory **46.** For example, authorized catheter types can be assigned validation codes, and the light system **1** will then only drive the catheter if it supplies a recognized validation code. This can beneficially prevent the use of unauthorized counterfeit catheters that may be substandard and present a health risk to the patient.

The catheter-side coupler **12** also includes a power supply **48** for power to be supplied thereto. In some examples, the power supply **48** can include a DC-to-DC converter which reduces the power supplied to the source-side coupler **10** from 12 Volts to 3.3 Volts. This is done in order to ensure that the source-side coupler **10** has plenty of power, even if the conductivity of the pins **34** degrades over time. In another embodiment, the electrical wires **22** can be configured to supply power to the fiber optic catheter apparatus **32.**

In other embodiments, the catheter-side electronic processor **40** is programmed to perform signal condition operations on a signal generated by the photodiode **36,** including peak detection, and on the temperature value measured by the temperature sensor **38.** The catheter-side electronic processor **40** can also include an analog-to-digital A/D) converter (not shown) in order to further process such signals.

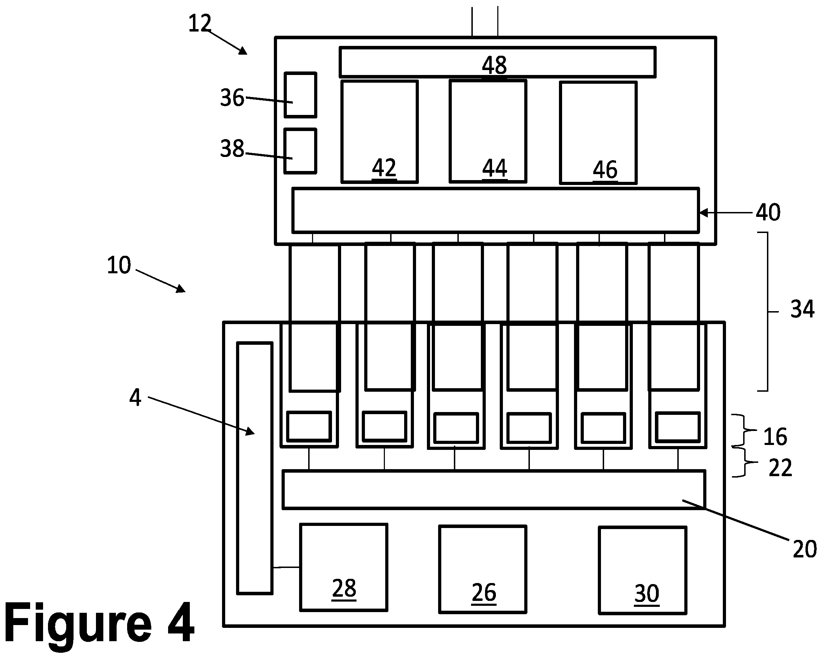

FIGURE 4 shows a connection between the source-side coupler **10** and the catheter-side coupler **12.** FIGURE 4 shows that the pins **34** of the catheter-side coupler **12** engage switches **16** of the source-side coupler **10.**

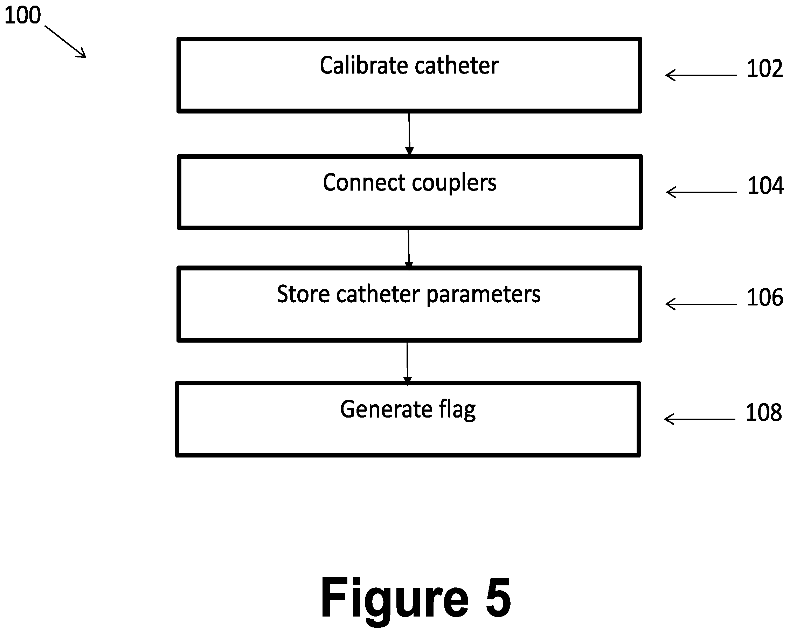

FIGURE 5 shows an example of a flowchart showing a catheter method **100** using the light system **1** and the fiber optic catheter apparatus **32.** At an operation **102,** one or more catheters **2** can be calibrated, and a calibration factor can be generated for each type of catheter and stored in the memory **46** of the catheter-side coupler **12.** Additionally, a unique catheter ID, min/max fluence, min/max rate, and run time parameters can be generated and stored. At an operation **104,** the catheter-side coupler **12** is connected to the source-side coupler **10** and a communication pathway is established via the serial communication circuits **30, 42.** At an operation **106,** the parameters stored in the memory **46** are transmitted to the source-side coupler **10.** In an optional operation **108,** one or more flags can be generated (e.g., a previous use flag, a technical issue flag, and so forth) and stored in the memory **36** during use of the catheter **2.**

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims.

## Claims

1. A source-side coupler **(10)** for injecting light from an associated light source **(4)** into an associated fiber optic catheter **(2)** via a connection of the source-side coupler with a catheter-side coupler **(12)** of the associated fiber optic catheter, the source-side coupler comprising:
an optical output aperture **(14)** configured to inject light from the associated light source into the associated fiber optic catheter via the connection;
a plurality of mechanical switches **(16)** configured to be engaged by pins **(34)** of the catheter-side coupler during the connection;
an electronic processor (20); and
at least two electrical lines **(22)** configured to establish an electrical connection between the electronic processor and the associated fiber optic catheter (2);
the electronic processor **(20)** being programmed to receive information from the associated fiber optic catheter via the connection by:
electrically receiving the information if the connection establishes an electrical connection between the electronic processor and the associated fiber optic catheter via the at least two electrical lines, or
mechanically receiving the information based on which mechanical switches of the plurality of mechanical switches are engaged by pins of the catheter-side coupler if the connection does not establish an electrical connection between the electronic processor and the associated fiber optic catheter via the at least two electrical lines.

2. The source-side coupler **(10)** of claim 1, wherein the electronic processor **(20)** is programmed to electrically receive the information via the at least two electrical lines **(22).**

3. The source-side coupler **(10)** of claim 1, further comprising a wireless radio receiver or transceiver **(26),** wherein the electronic processor **(20)** is programmed to electrically receive the information via the wireless radio receiver or transceiver.

4. A light system **(1)** for injecting light into an associated fiber optic catheter **(2),** the light system comprising:
a light source **(4);** and
a source-side coupler **(10)** as set forth in any one of claims 1-3.

5. A fiber optic catheter apparatus **(32),** comprising:
a catheter **(2)** including an optical fiber **(6)** or optical fiber bundle terminating at an optical output aperture **(8);**
a catheter-side coupler **(12)** including a plurality of electrically conductive pins **(34);** and
an electronic processor **(40)** programmed transmit information about the fiber optic catheter apparatus to an associated light source **(4)** in response to an electrical connection of the catheter-side coupler with a source-side coupler **(10)** of the associated light source via the electrically conductive pins;
wherein a physical configuration of the plurality of electrically conductive pins encodes at least a portion of the information about the fiber optic catheter apparatus.

6. The fiber optic catheter apparatus **(32)** of claim 5, wherein the electronic processor **(40)** is programmed to transmit the information about the fiber optic catheter apparatus to the associated light source **(4)** via the electrically conductive pins **(34).**

7. The fiber optic catheter apparatus **(32)** of claim 6, further comprising:
a wireless radio transmitter or transceiver **(44),** wherein the electronic processor **(40)** is programmed to transmit the information about the fiber optic catheter apparatus to the associated light source **(4)** via the wireless radio receiver or transceiver.

8. The fiber optic catheter apparatus **(32)** of any one of claims 5-7, wherein the electrically conductive pins **(34)** are configured to engage mechanical switches **(16)** of the source-side coupler **(10),** the mechanical switches including electrical wires **(22);** and
the electronic processor **(40)** is configured to detect a configuration of the electrically conductive pins that engage the mechanical switches via signals generated by the electrical wires of engaged mechanical switches to determine an identification of a catheter type of the fiber optic catheter apparatus.

9. The fiber optic catheter apparatus **(32)** of any one of claims 5-8, further including:
a memory **(46)** storing memory content including at least a catheter type of the fiber optic catheter apparatus;
wherein the transmitted information about the fiber optic catheter apparatus includes the memory content.

10. The fiber optic catheter apparatus **(32)** of claim 9, wherein the identification of the catheter type of the fiber optic catheter apparatus is transmitted to the memory **(46)** using a wired connection based on a number of mechanical switches **(16)** engaged by the electrically conducting pins **(34).**

11. The fiber optic catheter apparatus **(32)** of claim 10, wherein the electronic processor **(40)** is configured to generate a validation code indicative of whether the source-side coupler **(10)** has been validated for use, wherein the validation code is stored in the memory **(46).**

12. The fiber optic catheter apparatus **(32)** of any one of claims 5-11, further including:
a photodiode **(36)** configured to measure a power of a laser beam transmitted through the source-side coupler **(10).**

13. The fiber optic catheter apparatus **(32)** of claim 12, further including:
a temperature sensor **(38)** configured to measure an ambient temperature value of an ambient environment of the fiber optic catheter apparatus;
wherein the photodiode **(36)** is configured to measure the power of the laser beam based on the measured ambient temperature value.

14. The fiber optic catheter apparatus **(32)** of any one of claims 5-13, wherein the fiber optic catheter apparatus **(32)** is configured to receive power from electrical lines **(22)** of the source-side coupler **(10)** of the associated light source.

15. The light system **(1)** of claim 4 comprising the source side-coupler of claim 1, the light system comprising:
a fiber optic catheter **(32),** comprising:
a catheter **(2)** including an optical fiber **(6)** or optical fiber bundle terminating at an optical output aperture **(8);**
a catheter-side coupler **(12)** including a plurality of electrically conductive pins **(34);** and
a fiber optic catheter electronic processor **(40)** disposed in the catheter-side coupler and programmed transmit information about the fiber optic catheter apparatus to the light source;
wherein a physical configuration of the plurality of electrically conductive pins encodes at least a portion of the information about the fiber optic catheter apparatus;
wherein the plurality of mechanical switches of the source-side coupler are configured to be engaged by the electrically conductive pins of the catheter-side coupler during the connection.

## Patentansprüche

1. Quellenseitiger Koppler **(10)** zum Einkoppeln von Licht einer zugehörigen Lichtquelle **(4)** in einen zugehörigen Glasfaserkatheter **(2)** über eine Verbindung des quellenseitigen Kopplers mit einem katheterseitigen Koppler **(12)** des zugehörigen Glasfaserkatheters, wobei der quellenseitige Koppler Folgendes umfasst:
eine optische Ausgangsöffnung **(14),** die so konfiguriert ist, dass sie Licht von der zugehörigen Lichtquelle über die Verbindung in den zugehörigen Glasfaserkatheter einkoppelt;
eine Vielzahl von mechanischen Schaltern **(16),** die so konfiguriert sind, dass sie während des Verbindungsvorgangs mit Stiften **(34)** des katheterseitigen Kopplers in Eingriff kommen;
einen elektronischen Prozessor (20); und
mindestens zwei elektrische Leitungen (22), die so konfiguriert sind, dass sie eine elektrische Verbindung zwischen dem elektronischen Prozessor und dem zugehörigen Glasfaserkatheter (2) herstellen;
wobei der elektronische Prozessor **(20)** so programmiert ist, dass er über die Verbindung Informationen von dem zugehörigen Glasfaserkatheter empfängt, durch:
elektrisches Empfangen der Informationen, wenn die Verbindung eine elektrische Verbindung zwischen dem elektronischen Prozessor und dem zugehörigen Glasfaserkatheter über die mindestens zwei elektrischen Leitungen herstellt, oder
mechanisches Empfangen der Informationen, basierend auf welche mechanische Schalter der Vielzahl von mechanischen Schaltern mit Stiften des katheterseitigen Kopplers in Eingriff stehen, wenn die Verbindung keine elektrische Verbindung zwischen dem elektronischen Prozessor und dem zugehörigen Glasfaserkatheter über die mindestens zwei elektrischen Leitungen herstellt.

2. Quellenseitiger Koppler **(10)** nach Anspruch 1, wobei der elektronische Prozessor **(20)** so programmiert ist, dass er die Informationen über die mindestens zwei elektrischen Leitungen **(22)** elektrisch empfängt.

3. Quellenseitiger Koppler **(10)** nach Anspruch 1, ferner umfassend einen drahtlosen Funkempfänger oder Funksendeempfänger **(26),** wobei der elektronische Prozessor **(20)** so programmiert ist, dass er die Informationen elektrisch über den drahtlosen Funkempfänger oder Funksendeempfänger empfängt.

4. Lichtsystem **(1)** zum Einkoppeln von Licht in einen zugehörigen Glasfaserkatheter **(2),** wobei das Lichtsystem Folgendes umfasst:
eine Lichtquelle **(4);** und
einen quellenseitigen Koppler **(10)** nach einem der Ansprüche 1-3.

5. Glasfaserkathetereinrichtung **(32),** umfassend:
einen Katheter **(2),** der eine optische Faser **(6)** oder ein optisches Faserbündel einschließt, das an einer optischen Ausgangsöffnung **(8)** endet;
einen katheterseitigen Koppler **(12),** der eine Vielzahl elektrisch leitfähiger Stifte **(34)** einschließt; und
einen elektronischen Prozessor **(40),** der so programmiert ist, dass er Informationen über die Glasfaserkathetereinrichtung an eine zugehörige Lichtquelle **(4)** überträgt, sobald eine elektrische Verbindung zwischen dem katheterseitigen Koppler und einem quellenseitigen Koppler **(10)** der zugehörigen Lichtquelle über die elektrisch leitfähigen Stifte hergestellt wird;
wobei eine physikalische Konfiguration der Vielzahl elektrisch leitfähiger Stifte mindestens einen Teil der Informationen über die Glasfaserkathetereinrichtung kodiert.

6. Glasfaserkathetereinrichtung **(32)** nach Anspruch 5, wobei der elektronische Prozessor **(40)** so programmiert ist, dass er die Informationen über die Glasfaserkathetereinrichtung über die elektrisch leitfähigen Stifte **(34)** an die zugehörige Lichtquelle **(4)** überträgt.

7. Glasfaserkathetereinrichtung **(32)** nach Anspruch 6, ferner umfassend:
einen drahtlosen Funksender oder Funksendeempfänger **(44),** wobei der elektronische Prozessor **(40)** so programmiert ist, dass er die Informationen über die Glasfaserkathetereinrichtung über den drahtlosen Funkempfänger oder Funksendeempfänger an die zugehörige Lichtquelle **(4)** überträgt.

8. Glasfaserkathetereinrichtung **(32)** nach einem der Ansprüche 5-7, wobei die elektrisch leitfähigen Stifte **(34)** so konfiguriert sind, dass sie mit mechanischen Schaltern **(16)** des quellenseitigen Kopplers **(10)** in Eingriff kommen, wobei die mechanischen Schalter elektrische Drähte **(22)** einschließen; und
der elektronische Prozessor **(40)** so konfiguriert ist, dass er über Signale, die von den elektrischen Drähten von in Eingriff stehenden mechanischen Schaltern erzeugt werden, eine Konfiguration der elektrisch leitfähigen Stifte erfasst, die mit den mechanischen Schaltern in Eingriff stehen, um eine Identifizierung eines Kathetertyps der Glasfaserkathetereinrichtung zu bestimmen.

9. Glasfaserkathetereinrichtung **(32)** nach einem der Ansprüche 5-8, ferner einschließend:
einen Speicher **(46)** zur Speicherung von Speicherinhalten einschließlich mindestens eines Kathetertyps der Glasfaserkathetereinrichtung ;
wobei die übertragenen Informationen über die Glasfaserkathetereinrichtung den Speicherinhalt einschließen.

10. Glasfaserkathetereinrichtung **(32)** nach Anspruch 9, wobei die Identifizierung des Kathetertyps der Glasfaserkathetereinrichtung mittels einer Drahtverbindung, die auf einer Anzahl von mechanischen Schaltern **(16)** basiert, die mit den elektrisch leitenden Stiften **(34)** in Eingriff stehen, an den Speicher **(46)** übertragen wird.

11. Glasfaserkathetereinrichtung **(32)** nach Anspruch 10, wobei der elektronische Prozessor **(40)** so konfiguriert ist, dass er einen Validierungscode generiert, der angibt, ob der quellenseitige Koppler **(10)** für die Verwendung validiert wurde, wobei der Validierungscode in dem Speicher **(46)** gespeichert ist.

12. Glasfaserkathetereinrichtung **(32)** nach einem der Ansprüche 5-11, ferner einschließend:
eine Fotodiode **(36),** die so konfiguriert ist, dass sie die Leistung eines durch den quellenseitigen Koppler **(10)** übertragenen Laserstrahls misst.

13. Glasfaserkathetereinrichtung **(32)** nach Anspruch 12, ferner einschließend:
einen Temperatursensor **(38),** der so konfiguriert ist, dass er einen Umgebungstemperaturwert einer Umgebung der Glasfaserkathetereinrichtung misst;
wobei die Fotodiode **(36)** so konfiguriert ist, dass sie die Leistung des Laserstrahls auf der Grundlage des gemessenen Umgebungstemperaturwertes misst.

14. Glasfaserkathetereinrichtung **(32)** nach einem der Ansprüche 5-13, wobei die Glasfaserkathetereinrichtung **(32)** so konfiguriert ist, dass sie Energie von elektrischen Leitungen **(22)** des quellenseitigen Kopplers **(10)** der zugehörigen Lichtquelle empfängt.

15. Lichtsystem **(1)** nach Anspruch 4, umfassend den quellenseitigen Koppler nach Anspruch 1, wobei das Lichtsystem Folgendes umfasst:
einen Glasfaserkatheter **(32),** umfassend:
einen Katheter **(2),** der eine optische Faser **(6)** oder ein optisches Faserbündel einschließt, das an einer optischen Ausgangsöffnung **(8)** endet;
einen katheterseitigen Koppler **(12),** der eine Vielzahl elektrisch leitfähiger Stifte **(34)** einschließt; und
einen elektronischen Prozessor **(40)** für den Glasfaserkatheter, der im katheterseitigen Koppler angeordnet ist und so programmiert ist, dass er Informationen über die Glasfaserkathetereinrichtung an die Lichtquelle überträgt;
wobei eine physikalische Konfiguration der Vielzahl elektrisch leitfähiger Stifte mindestens einen Teil der Informationen über die Glasfaserkathetereinrichtung kodiert;
wobei die Vielzahl mechanischer Schalter des quellenseitigen Kopplers so konfiguriert ist, dass sie während des Verbindungsvorgangs mit den elektrisch leitfähigen Stiften des katheterseitigen Kopplers in Eingriff kommt.

## Revendications

1. Coupleur côté source **(10)** pour injecter de la lumière provenant d'une source de lumière **(4)** associée dans un cathéter à fibres optiques **(2)** associé via une connexion entre le coupleur côté source et un coupleur côté cathéter **(12)** du cathéter à fibres optiques associé, le coupleur côté source comprenant :
une ouverture de sortie optique **(14)** configurée pour injecter de la lumière provenant de la source de lumière associée dans le cathéter à fibres optiques associé via la connexion ;
une pluralité de commutateurs mécaniques **(16)** configurés pour venir en prise avec des broches **(34)** du coupleur côté cathéter pendant la connexion ;
un processeur électronique (20) ; et
au moins deux lignes électriques (22) configurées pour établir une connexion électrique entre le processeur électronique et le cathéter à fibres optiques (2) associé ;
le processeur électronique **(20)** étant programmé pour recevoir des informations provenant du cathéter à fibres optiques associé via la connexion par :
la réception électrique des informations si la connexion établit une connexion électrique entre le processeur électronique et le cathéter à fibres optiques associé via les au moins deux lignes électriques, ou
la réception mécanique des informations sur lesquelles des commutateurs mécaniques de la pluralité de commutateurs mécaniques viennent en prise avec les broches du coupleur côté cathéter si la connexion n'établit pas de connexion électrique entre le processeur électronique et le cathéter à fibres optiques associé via les au moins deux lignes électriques.

2. Coupleur côté source **(10)** selon la revendication 1, dans lequel le processeur électronique **(20)** est programmé pour recevoir électriquement l'information via les au moins deux lignes électriques **(22).**

3. Coupleur côté source **(10)** selon la revendication 1, comprenant en outre un récepteur ou émetteur-récepteur radio sans fil **(26),** dans lequel le processeur électronique **(20)** est programmé pour recevoir électriquement les informations via le récepteur ou émetteur-récepteur radio sans fil.

4. Système d'éclairage **(1)** pour injecter de la lumière dans un cathéter à fibres optiques **(2)** associé, le système d'éclairage comprenant :
une source de lumière **(4)** ; et
un coupleur côté source **(10)** tel que décrit selon l'une quelconque des revendications 1-3.

5. Appareil de cathéter à fibres optiques **(32),** comprenant :
un cathéter **(2)** incluant une fibre optique **(6)** ou un faisceau de fibres optiques se terminant par une ouverture de sortie optique **(8) ;**
un coupleur côté cathéter **(12)** incluant une pluralité de broches électroconductrices **(34)** ; et
un processeur électronique **(40)** programmé pour transmettre des informations sur l'appareil de cathéter à fibres optiques à une source de lumière **(4)** associée en réponse à une connexion électrique du coupleur côté cathéter avec un coupleur côté source **(10)** de la source de lumière associée via les broches électroconductrices ;
dans lequel une configuration physique de la pluralité de broches électroconductrices code au moins une partie des informations concernant l'appareil de cathéter à fibres optiques.

6. Appareil de cathéter à fibres optiques **(32)** selon la revendication 5, dans lequel le processeur électronique **(40)** est programmé pour transmettre les informations sur l'appareil de cathéter à fibres optiques à la source de lumière **(4)** associée via les broches électroconductrices **(34).**

7. Appareil de cathéter à fibres optiques **(32)** selon la revendication 6, comprenant en outre :
un émetteur ou émetteur-récepteur radio sans fil **(44),** dans lequel le processeur électronique **(40)** est programmé pour transmettre les informations sur l'appareil de cathéter à fibres optiques à la source de lumière **(4)** associée via le récepteur ou émetteur-récepteur radio sans fil.

8. Appareil de cathéter à fibres optiques **(32)** selon l'une quelconque des revendications 5-7, dans lequel les broches électroconductrices **(34)** sont configurées pour venir en prise avec des commutateurs mécaniques **(16)** du coupleur côté source **(10),** les commutateurs mécaniques incluant des fils électriques **(22)** ; et
le processeur électronique **(40)** est configuré pour détecter une configuration des broches électroconductrices qui viennent en prise avec les commutateurs mécaniques via des signaux générés par les fils électriques des commutateurs mécaniques mis en prise afin de déterminer une identification du type de cathéter de l'appareil de cathéter à fibres optiques.

9. Appareil de cathéter à fibres optiques **(32)** selon l'une quelconque des revendications 5-8, incluant en outre :
une mémoire **(46)** stockant un contenu de mémoire incluant au moins un type de cathéter de l'appareil de cathéter à fibres optiques ;
dans lequel les informations transmises concernant l'appareil de cathéter à fibres optiques incluent le contenu de mémoire.

10. Appareil de cathéter à fibres optiques **(32)** selon la revendication 9, dans lequel l'identification du type de cathéter de l'appareil de cathéter à fibres optiques est transmise à la mémoire **(46)** à l'aide d'une connexion filaire basée sur un certain nombre de commutateurs mécaniques **(16)** mis en prise avec les broches électroconductrices **(34).**

11. Appareil de cathéter à fibres optiques **(32)** selon la revendication 10, dans lequel le processeur électronique **(40)** est configuré pour générer un code de validation indiquant si le coupleur côté source **(10)** a été validé pour utilisation, dans lequel le code de validation est stocké dans la mémoire **(46).**

12. Appareil de cathéter à fibres optiques **(32)** selon l'une quelconque des revendications 5-11, incluant en outre :
une photodiode **(36)** configurée pour mesurer la puissance d'un faisceau laser transmis à travers le coupleur côté source **(10).**

13. Appareil de cathéter à fibres optiques **(32)** selon la revendication 12, incluant en outre :
un capteur de température **(38)** configuré pour mesurer la valeur de température ambiante de l'environnement ambiant de l'appareil de cathéter à fibres optiques ;
dans lequel la photodiode **(36)** est configurée pour mesurer la puissance du faisceau laser en fonction de la valeur de température ambiante mesurée.

14. Appareil de cathéter à fibres optiques **(32)** selon l'une quelconque des revendications 5-13, dans lequel l'appareil de cathéter à fibres optiques **(32)** est configuré pour recevoir la puissance des lignes électriques **(22)** du coupleur côté source **(10)** de la source de lumière associée.

15. Système d'éclairage **(1)** selon la revendication 4 comprenant le coupleur côté source selon la revendication 1, le système d'éclairage comprenant :
un cathéter à fibres optiques **(32),** comprenant :
un cathéter **(2)** incluant une fibre optique **(6)** ou un faisceau de fibres optiques se terminant par une ouverture de sortie optique **(8)** ;
un coupleur côté cathéter **(12)** incluant une pluralité de broches électroconductrices **(34)** ; et
un processeur électronique de cathéter à fibres optiques **(40)** disposé dans le coupleur côté cathéter et programmé pour transmettre des informations sur l'appareil de cathéter à fibres optiques à la source de lumière ;
dans lequel une configuration physique de la pluralité de broches électroconductrices code au moins une partie des informations concernant l'appareil de cathéter à fibres optiques ;
dans lequel la pluralité de commutateurs mécaniques du coupleur côté source sont configurés pour être mis en prise avec les broches électroconductrices du coupleur côté cathéter pendant la connexion.
